# EUROPEAN PATENT APPLICATION

(11) **EP 2 583 669 A1**
(43) Date of publication of application: **24.04.2013**
(21) Application number: 13000223.1
(22) Date of filing: 10.10.2008
(51) Int. Cl.: A61K 9/00, A61K 9/20, A61K 31/13, A61K 47/48, A61K 9/16, A61K 9/50

(54) **Taste-masked orally disintegrating tablets of memantine hydrochloride**

(30) Priority: 10.10.2007 IN MU20242007
(62) Divisional of application: 08869072.2
(71) Applicant: Rubicon Research Private Limited, Mumbai 400 078 (IN)
(72) Inventor: Pilgaonkar, Pratibha S., 400 078 Mumbai (IN); Rustomjee, Maharukh T., 400 078 Mumbai (IN); Gandhi, Anilkumar S., 400 078 Mumbai (IN)
(74) Representative: Schütte, Hartmut

(57) **Abstract**

The present invention relates to a solid pharmaceutical composition comprising memantine, which dissolves or disintegrates in the oral cavity preferably within about 60 seconds. The present invention further discloses orally disintegrating tablets of taste-masked memantine of optimal mechanical strength comprising memantine along with a taste-masking agent and pharmaceutically acceptable excipients.

## Description

### Field of the Invention

The present invention relates to a solid pharmaceutical composition comprising memantine, which dissolves or disintegrates in oral cavity, preferably within about 60 seconds.

The present invention also relates to a process for the preparation of a taste-masked pharmaceutical composition of memantine of optimal mechanical strength comprising memantine along with a taste-masking agent and at least one pharmaceutically acceptable excipient.

### Background of the Invention

The geriatric population composed of people ages 65 years and over, is rapidly growing in the United States and throughout the world. According to the United Nations World Prospects published in 2005, the global population of people over age 60 years in 2005 was estimated to be 672 million out of a total of approximately 6.5 billion and is expected to reach 1.9 billion by 2050 (cited in: Population Division of the Department of Economic and Social Affairs of the United Nations Secretariat. The World Population Prospects: the 2004 revision). This population presents unique treatment challenges for caregivers and physicians. The prevalence of psychiatric illness ranges from 65-90%, with dementia complicated by depression, psychosis, and behavioral disturbances, the most common disorder. Some of them may also suffer from chronic mental illnesses including schizophrenia, bipolar disorder, major depression, panic disorder, posttraumatic stress disorder, and mental retardation. All these diseases further complicate the administration of a medication.

Further, the pediatric populations, especially the ones with childhood neurodevelopmental disorders like autism and attention deficit hyperactivity syndrome, have a compromised quality of life and present a unique challenge to caregivers in terms of ease of administration, patient comfort and compliance with the prescribed dose regimen.

Most commonly employed pharmaceutical preparations, such as tablets or liquid orals, are not suitable for these populations. They may have difficulty in swallowing the dosage form and with liquids administration; stability of the dosage form and drug can be a major concern. An ideal dosage form for these patients includes a formulation that simply melts in the mouth without any extra efforts. Several orally disintegrating tablets have therefore been specifically prepared for drugs employed for old age disorders such as Parkinson or Alzheimer's disease or childhood disorders such as autism or attention deficit hyperactivity syndrome.

Memantine hydrochloride, 1-amino-3,5-dimethyladamantane hydrochloride provides good and persistent activation of central nervous N-methyl-D-aspartate (NMDA) receptors and is used to treat Alzheimer's disease and other dementias, usually for elderly patients.

Memantine is the first and only representative of a new class of Alzheimer drugs - a moderate affinity NMDA-receptor antagonist. Memantine was developed by Merz and licensed to Forest for the U.S. and Lundbeck for selected European and international markets. Memantine is marketed under the brands **Axura**® and **Akatinol**® by Merz, **Namenda**® by Forest and **Ebixa**® by Lundbeck for the treatment of moderate to severe Alzheimer's disease. Clinical data on memantine show that memantine improves cognitive and psychomotor functioning, improves activities of daily living leading to reduction of care dependence and has excellent tolerability. It produces symptomatic improvements in learning under conditions of tonic NMDA receptor activation in Alzheimer's disease. In contrast to first generation therapies, memantine is likely to show neuroprotective effects at concentrations used in the treatment of Alzheimer's disease and to slow down disease progression. It is available as tablets 5 and 10 mg and as an oral solution (2 mg/ml). Since memantine is indicated for Alzheimer's disease that occurs in elderly people, there is a need for dosage forms which can be administered to these subjects with minimum difficulty. Such novel dosage forms like orally disintegrating tablets will also significantly improve the compliance of elderly patients.

United States Patent No. 5,382,601 describes solid pharmaceutical dosage forms containing memantine, which exhibit an extended two-phase release profile, with a portion of the drug being released immediately, followed by a sustained release of the remainder. The matrix of this formulation contains both a water-soluble and a water-insoluble salt of casein, preferably sodium and calcium caseinate.

United States Patent No. 6,194,000 discloses a method of preparing modified release N-methyl-D-aspartate (NMDA) receptor antagonists comprising preparing an instant release component and a modified release component to arrive at the final formulation. The patent discloses the formulations consisting of encapsulated beads previously coated using organic solvent-based systems.

U.S. Patent Application US2006/0198884A1 discloses immediate release formulations containing 1-aminocyclohexanes, preferably memantine or neramexane, and optionally a pharmaceutically acceptable coating, wherein the active ingredient exhibits dose proportionality and is released at a dissolution rate of more than about 80% within about the first 60 minutes. The dosage form is directly compressed and allows for dose-proportional compositions for once or twice daily dosing. U.S. Patent Application US2006/0002999 also discloses immediate release dosage formulations of memantine. Further, PCT Application WO2006/009769, U.S. Patent Application 2004/0254251, and U.S. Patent Application 2006/0051416 describe modified release formulations of memantine.

Although several attempts have been made to develop immediate as well as controlled release formulations of memantine, the prior art does not disclose a rapidly disintegrating oral formulation of memantine. Memantine is indicated for conditions prevalent in geriatric patients and can also be beneficial in conditions of childhood behavioral disorders and therefore there is a need for development of formulations that increase compliance of geriatric and pediatric patient population. A liquid formulation of memantine is available; however the liquid dosage form itself has its own limitations. The bitter taste of memantine is another reason for developing alternate oral formulations of memantine. Thus there exists a need for taste-masked, patient-friendly dosage forms such as dispersible tablets or orally disintegrating tablets of memantine.

The present invention addresses this need and discloses, after rigorous experimentation, taste-masked memantine formulations that rapidly disintegrate in the mouth. These tablet compositions have a pleasant mouth feel and good mechanical strength. These tablet compositions are robust (e.g., low friability, low ejection forces, hardness) enough to be processed in high speed tableting machines and shipped in low cost packages, and at the same time disintegrate in less than 60 seconds in the oral cavity.

### Summary of the Invention

The present invention provides taste-masked orally disintegrating tablet compositions of memantine which have optimal mechanical strength and *in vitro* disintegration time of about 60 seconds. The compositions of the present invention comprise:
a. taste-masked memantine; and
b. at least one pharmaceutically acceptable excipient.

### Detailed Description of the Invention

The present invention provides a taste-masked pharmaceutical composition of memantine which dissolves or disintegrates in oral cavity without water, preferably within about 60 seconds. The present invention provides an orally disintegrating tablet composition of memantine or pharmaceutically acceptable salt thereof comprising (a) memantine or pharmaceutically acceptable salts thereof (b) at least one taste-masking agent; and (c) at least one pharmaceutically acceptable excipient. The bitter taste of memantine of the composition of the present invention is masked by coating (such as using water insoluble coatings), complexation (such as with ion exchange resins), salt formation (such as with sodium saccharin), use sweeteners or flavors and the like, or any combination thereof. Taste-masking agent incorporated in the compositions of the present invention is ion exchange resin, water insoluble excipient, cyclodextrin, sugar substitute for salt formation, sweetener, flavor, or any combination thereof.

The present invention provides orally disintegrating tablet compositions of taste-masked memantine using coprocessed excipients. The present invention further provides orally disintegrating tablet compositions of taste-masked memantine which retard the release of memantine.

The term 'memantine' and 'memantine hydrochloride' are used interchangeably for the purpose of this invention. Memantine may preferably be used in the form of a pharmaceutically acceptable salt. Further, suitable salts include, but are not limited to, acid addition salts, such as those made with hydrochloric, hydrobromic, hydroiodic, methylsulfonic, perchloric, sulfuric, nitric, phosphoric, acetic, propionic, glycolic, lactic pyruvic, malonic, succinic, maleic, fumaric, maleic, tartaric, citric, benzoic, carbonic cinnamic, mandelic, methanesulfonic, ethanesulfonic, benezenesulfonic, hydroxyethanesulfonic, p-toluene sulfonic, cyclohexanesulfamic, salicyclic, p-aminosalicylic, 2-phenoxybenzoic, and 2-acetoxy benzoic acid. The term "salt" or "salts" also include addition salts of free acids or free bases. All such salts are acceptable provided that they are non-toxic and do not substantially interfere with the desired pharmacological activity. Additionally, polymorphs, hydrates and solvates as well as amorphous forms of memantine can be utilized for the purpose of the present invention. Thus the term "memantine" as used in the present invention encompasses both the free base and pharmaceutically acceptable salts thereof. In some embodiments of the present invention, the active ingredient is memantine hydrochloride.

The tablet compositions of the present invention include about 0.5% to about 60% by weight of memantine. In one embodiment of the present invention, the compositions of the present invention include about 1% to about 35% by weight of memantine.

The present invention is also applicable to combinations of memantine with other drugs. In one embodiment of the present invention, taste-masked orally disintegrating compositions of the present invention comprise memantine or an analog thereof in combination with other active agents selected from, but not limited to, neramaxane or other 1-aminocyclohexane derivatives, donepezil, rivastigmine, metrifonate, tacrine, amantadine, galantamine, olanzapine, piracetam, aniracetam, nefiracetam, oxiracetam, lazabemide, selegiline, amitriptyline, clomipramine, trimipramine, doxepin, nortriptyline, desipramine, protriptyline, fluoxetine, paroxetine, citalopram, sertraline, fluvoxamine, escitalopram, reboxetine, atomoxetine, venlafaxine, milnacipran, and duloxetine.

Orally disintegrating tablets (ODTs) disintegrate/dissolve in the mouth rapidly without administering extra water, providing the convenience of a tablet formulation while allowing the ease of swallowing provided by a liquid formulation. Such dosage forms due to their ease of administration and pleasant mouth feel may encourage patients especially the elderly patients and children who have difficulty in swallowing conventional tablets to adhere to daily medication regimens and also allow the luxury of much more accurate dosing than oral liquids. Such tablets also serve useful where water may not be readily available to assist in swallowing the tablet in specific conditions or patient is not in a condition to use water and swallow the conventional tablet.

The bitter taste of memantine poses a great challenge for the development of orally disintegrating tablet formulations. The present invention employs a number of approaches including, but not limited to, using coating, complexation, salt formation, sweeteners, flavours and the like for masking of the unpleasant taste of memantine.

Coating is the most widely used technique for taste masking of pharmaceuticals .This can be achieved using polymers or lipids to mask the unpleasant taste. In an embodiment of the present invention, memantine is taste masked by coating with water insoluble excipients. The term "water insoluble excipient" as used herein includes polymeric or non-polymeric materials that are not completely soluble in water across pH range of 1-8 commonly encountered in gastrointestinal tract or are only partially soluble at, at least a particular pH.

Polymeric water-insoluble excipients for taste masking memantine as employed in the present invention include, but are not limited to, vinyl acetate, vinyl chloride, vinyl carbonate, methacrylic acid, a polymethacrylic acid copolymer, other polymethylmethacrylates, ethyl cellulose, nitrocellulose, vinylidene chloride-acrylonitrile copolymer, acrylonitrile-styrene copolymer, polyethylene, polyethylene oxide, polystyrene, ethylene vinyl acetate, cellulose acetate, cellulose acetate phthalate, cellulose acetate butyrate, copolymers of vinyl pyrrolidone, and hydroxypropylmethylcellulose phthalate or any mixtures thereof.

Non-polymeric water insoluble excipients for taste masking memantine as employed in the present invention include, but are not limited to, waxes, fatty acids and long chain monohydric alcohols. Waxes include, but are not limited to, Cutina (hydrogenated castor oil), Hydrobase (hydrogenated soybean oil), Castorwax (hydrogenated castor oil), Croduret (hydrogenated castor oil), Carbowax, Compritol (glyceryl behenate), Sterotex (hydrogenated cottonseed oil), Lubritab (hydrogenated cottonseed oil), Apifil (wax yellow), Akofine (hydrogenated cottonseed oil), Softtisan (hydrogenated palm oil), Hydrocote (hydrogenated soybean oil), Corona (lanolin), Gelucire (macrogolglycerides lauriques), Precirol (glyceryl palmitostearate), Emulcire (cetyl alcohol), Plurol diisostearique (polyglyceryl diisostearate), and Geleol (glyceryl stearate), and mixtures thereof. The fatty acids include, but are not limited to, hydrogenated palm oil, hydrogenated castor oil, hydrogenated cottonseed oil, stearic acid, and palmitic acid, and mixtures thereof. Long chain monohydric alcohols include, but are not limited to, cetyl alcohol, and stearyl alcohol and mixtures thereof.

Water-insoluble excipients of the present invention can also be employed in combination with water soluble excipients for taste-masking memantine. Water soluble excipients of the present invention inlcude, but are not limited to, polyvinylpyrrolidone (PVP), hydroxypropylmethylcellulose (HPMC), polyethylene glycol (PEG), poloxamer, hydroxypropyl cellulose (HPC), methylcellulose, guar gum, xanthan gum, gum arabic, tragacanthan, lactose, mannitol and hydroxyethyl cellulose, or any mixtures thereof.

Coating of the active ingredient can be done by any of the techniques known in the art like microencapsulation, hot melt granulation, fluid bed coating, wet granulation or spray drying. Further, the taste masking coatings, based on lipids or waxes require that the melting point of the lipid should be sufficiently high to prevent melting in the mouth and should not be so high that active ingredient itself melts or is chemically degraded while preparation of the formulation. Lipids or waxes can also be employed in the form of an aqueous dispersion stabilized by surfactants and suitable stabilizers. Coating can be carried out in the range from about 1% to about 60% by weight of the memantine composition.

Ion exchange resins are usually made from a polymer backbone with various displaceable functional groups ionically bonded to the polymer. The polymer chains are also typically cross linked, leading to a gel-like insoluble composition formed in beads. Cationic ion exchange resins have negatively charged or anionic, binding sites. The anionic binding sites are bonded to displaceable cationic groups. Memantine hydrochloride is positively charged and displaces the cationic groups, typically becoming bonded to the resin by ionic bonds. Various ion exchange resins that can be employed for taste masking of memantine include Amberlite IRP 64, IRP-69 and IRP-70 (copolymer of methacrylic acid crosslinked with divinylbenzene which differ in particle size) and Dowex (based on polystyrenesulfonic acid crosslinked with divinylbenzene). These resins are employed in weight ratio of 1:0.1 to 1:20 with respect to memantine.

Along with ion exchange resins, several cyclodextrin derivatives are also employed for complexation and hence taste-masking of memantine hydrochloride. Cyclodextrins are crystalline, cyclic oligosaccharides derived from starch. Examples of cyclodextrin derivatives which are employed for memantine taste-masking include hydroxy propyl β-Cyclodextrin and 7-suphobutyl ether of cyclodextrin. Cyclodextrin makes an inclusion complex with the memantine molecule by acting as a hydrophobic host cavity. Such complexes for taste-masking purposes can be processed by grinding, solid dispersion, kneading, spray drying and melting method.

Taste-masking of memantine can also be achieved by formation of salt with equimolar amounts of sugar substitutes like cyclamate, saccharin, acesulfame or a mixture of at least two of the sugar substitutes. This results in formation of taste-masked memantine salt. The memantine salt has reduced bitter taste and thus results in improved patient compliance. Such a taste-masked salt can be compressed in the form of a tablet.

Masking of the bitter taste of memantine is also achieved using at least one sweetening agent such as, but not limited to, aspartame, stevia extract, glycyrrhiza, saccharine, saccharine sodium, acesulfame, sucralose and dipotassium glycyrrhizinate; and one or more flavors, *e.g.*, mint flavour, orange flavour, lemon flavors, strawberry aroma, vanilla flavour, raspberry aroma, cherry flavor, tutty frutty flavor,magnasweet 135, key lime flavor, grape flavor, trusil art 511815, and fruit extracts.

The active ingredient may be incorporated in the formulation in the powder form, granules, pellets, beads or any other suitable form. The taste-masking formulations may be so designed that the bioavailability of the memantine is preferably not compromised.

Design of an orally disintegrating tablet requires a significant amount of research work in order to develop a process that maintains enough porosity inside the compressed tablets for fast dissolving or fast melting while maintaining the mechanical strength of the tablet. Orally disintegrating tablets can be prepared by any of the known non limiting techniques such as freeze-drying, molding and sublimation, compression, cotton candy process, mass extrusion, etc or use of specialized excipients such as effervescent couple, highly micronized agents, coprocessed excipients or the like.

Directly compressible excipient employed in the present invention is a coprocessed excipient which is as described in PCT Application WO2007052289 and is incorporated herein by reference. This coprocessed excipient comprise of at least one water soluble excipient and water insoluble inorganic excipient such as calcium silicate. The water soluble carbohydrate can be a monosaccharide, disaccharide, oligosaccharide or polysaccharide. Examples of carbohydrates include, but are not limited to, monosaccharides such as sorbitol, glucose, dextrose, fructose, maltose or xylitol, disaccharides such as sucrose, trehalose, lactose, glucose, galactose or mannitol, and oligosaccharides and polysaccharides such as dextrates and maltodextrins. The water soluble and water insoluble excipients in the directly compressible composite can be in a ratio of water-soluble excipient to water insoluble excipient of from about 50:1 to about 1:50. In one embodiment of the present invention, this ratio is about 30:1 to about 1:30. In a further embodiment of the present invention, this ratio is from about 20:1 to about 1:20. The amount of directly compressible coprocessed excipient employed in the composition is about 5% to about 95 % by weight of the said dosage form. The term 'coprocessed excipient', 'composite excipient' and 'directly compressible excipient' has been employed interchangeably for the purpose of this invention.

The tablet composition of the present invention may include in addition to the taste-masked active ingredient and directly compressible coprocessed excipient, one or more binders, disintegrants, superdisintegrants, diluents, salivating agents, surfactants, flavors, sweeteners, colorants, diluents, souring agents, viscosity builders, glidants or lubricants, solubilizers, or stabilizers.

The tablet compositions of the present invention include at least one superdisintegrant such as, but not limited to, natural, modified or pregelatinized starch, crospovidone, croscarmellose sodium, sodium starch glycolate, low-substituted hydroxypropyl cellulose as well as effervescent disintegrating systems. In one embodiment, the disintegrants include crospovidone and starch. The amount of superdisintegrant employed in the composition is about 2% to about 30 % by weight of the said dosage form.

Examples of suitable binders include, but are not limited to, starch, pregelatinized starch, polyvinyl pyrrolidone (PVP), Copovidone, cellulose derivatives, such as hydroxypropylmethyl cellulose (HPMC), hydroxypropyl cellulose (HPC) and carboxymethyl cellulose (CMC) and their salts. Examples of suitable diluents include, but are not limited to, starch, microcrystalline cellulose, lactose, xylitol, mannitol, dicalcium phosphate, and the like.

Examples of the lubricant include, but are not limited to, magnesium stearate, calcium stearate, stearic acid, talc, and sodium stearyl fumarate. The tablet compositions of the invention may also include a glidant such as, but not limited to, colloidal silica, silica gel, precipitated silica, or combinations thereof.

The said compositions may also include salivating agents such as, but not limited to, micronised polyethylene glycol, sodium chloride or precipitated micronised silica to improve the disintegration properties of the said compositions.

Examples of solubilizers include, but are not limited to cetostearyl alcohol, cholesterol, diethanolamine, ethyl oleate, ethylene glycol palmitostearate, glycerin, glyceryl monostearate, isopropyl myristate, lecithin, medium-chain glyceride, monoethanolamine, oleic acid, propylene glycol, polyoxyethylene alkyl ether, polyoxyethylene castor oil glycoside, polyethylene sorbitan fatty acid ester, polyoxyethylene stearate, propylene glycol alginate, sorbitan fatty acid ester, stearic acid, sunflower oil, triethanolmine, and mixtures thereof. The tablet compositions of the present invention may also include stabilizers such as, but not limited to, benzoic acid, sodium benzoate, citric acid, and the like. Examples of surfactants include, but are not limited to, sodium docusate, glyceryl monooleate, polyethylene alkyl ether, polyoxyethylene sorbitan fatty acid ester, sodium lauryl sulfate, sorbic acid, sorbitan fatty acid ester, and mixtures thereof.

Souring agents include, but are not limited to, monosodium fumarate and/or citric acid. The tablet compositions of the present invention may optionally include viscosity building agents such as polyalkylene oxides; polyols; starch and starch-based polymers; chitosan; polysaccharide gums; polyethylene oxide, hydroxypropyl cellulose, hydroxypropyl methyl cellulose, hydroxyethyl cellulose, sodium carboxy methylcellulose, calcium carboxymethyl cellulose, methyl cellulose, polyacrylic acid, gum acacia, gum tragacanth, xanthan gum, guar gum and polyvinyl alcohol and copolymers and mixtures thereof.

Orally disintegrating tablets as described in the present invention are immediate release dosage forms and that release the taste masked memantine instantly upon reaching either stomach or intestine. In one embodiment of the present invention, the orally disintegrating tablets of the present invention comprise a composition wherein taste-masked memantine is released in a controlled manner over a period of time, for example, from about 2 to about 24 hours. In such a formulation, taste-masked memantine is further coated with a combination of water soluble and water insoluble excipients, as discussed above. The amount of coating not only ensures the taste-masking but also controls the release of taste-masked memantine. Any of the taste-masking approaches described above can be used in combination with a coating agent for the purpose of achieving a controlled release orally disintegrating tablets of memantine.

In another embodiment of the present invention, pellets or granules or the like of memantine are prepared comprising at least one release retardant in combination with one or more pharmaceutically acceptable excipients. Suitable release retardants include, but are not limited to, cellulose ethers, such as hydroxypropylmethylcellulose (HPMC), hydroxypropylcellulose (HPC), hydroxyethylcellulose and carboxymethylcellulose sodium); polysaccharides, such as carageenan, guar gum, xanthan gum, tragacanth and ceratonia; polymethacrylates, such as copolymers of acrylic and methacrylic acid esters containing quarternary ammonium groups; cellulose esters, such as cellulose acetate; acrylic acid polymers, such as carbomers; waxes, such as hydrogenated castor oil, hydrogenated vegetable oil, carnauba wax and microcrystalline wax; alginates, such as alginic acid and sodium alginate; and fatty acid derivatives, such as glyceryl monostearate and glyceryl palmitostearate. These pellets or granules or the like are further coated using excipients described above in order to achieve taste-masking of memantine.

The terms "solid dosage form," "tablet," "tablet composition" and "solid preparation" are used synonymously within the context of the present invention. These terms should be construed to include a compacted or compressed powder composition obtained by compressing or otherwise forming the composition to form a solid having a defined shape. Tablets in accordance with the invention may be manufactured using conventional techniques of common tableting methods known in the art such as direct compression, wet granulation, dry granulation and extrusion/ melt granulation. In one embodiment, the process is direct compression which involves compression of taste-masked drug-excipient blend after mixing them for a definite time period.

The tablet may vary in shape such as oval, triangle, almond, peanut, parallelogram, round, pentagonal, hexagonal, and trapezoidal. The preferred shapes are round, oval and parallelogram forms.

The performance of the orally disintegrating tablets can be evaluated using a number of parameters namely wicking time, mouth dissolution time, in vitro disintegration time, etc. The term 'wicking time' as used here provides time (seconds) taken for water to wick into the tablet and completely wet the tablet core. The term 'mouth dissolution time' as used herein provides time (seconds) taken for tablet to completely dissolve/disintegrate in the mouth determined in and by human volunteers. The term 'in vitro disintegration time' as used herein refers to the time taken for complete disintegration of the tablet as determined using the USP disintegration apparatus. As per various embodiments of the present invention, in vitro disintegration time is less than 60 seconds.

While the present invention has been described in terms of its specific embodiments, certain modifications and equivalents will be apparent to those skilled in the art and are intended to be included within the scope of the present invention. Details of the present invention, including its objects and advantages, are provided in the non-limiting exemplary illustrations below.

### EXAMPLES

### Example 1: Taste-masking of memantine using Eudragit EPO

**Table 1: Composition of orally disintegrating tablets**

| **Ingredients** | **mg/ tablet** |
|---|---|
| Memantine hydrochloride | 10.0 |
| Eudragit EPO,USP | 10.0 |
| Directly compressible excipient comprising mannitol and calcium silicate | 75.0 |
| Starch, Ph.Eur. | 12.0 |
| Microcrystalline cellulose, USP | 28.0 |
| Crospovidone, USP | 15.0 |
| Aspartame, USP | 3.0 |
| Acesulfame K, Ph.Eur. | 3.0 |
| Colloidal silicon dioxide, USP | 1.5 |
| Grapes Flavor | 1.0 |
| Magnesium stearate, USP | 1.5 |
| **Total** | **160.0** |

Memantine was dry mixed with Eudragit EPO and granulated using water. These granules were dried and then blended with other excipients except lubricant to get a uniform mass. The mass was lubricated and compressed into tablets having following parameters:
Hardness (N) : 20-40
Friability (%) : 0.38 %
Disintegration time (sec) : 10-15
Disintegration time in oral cavity (sec) : 25-30

Tablets with desired taste masking, friability and disintegration time were obtained.

### Example 2: Taste-masking of memantine using ion exchange resins

**Table 2: Composition of orally disintegrating tablets**

| **Ingredients** | **mg/ tablet** |
|---|---|
| Memantine hydrochloride | 5.0 |
| Cationic ion exchange resin | 10.0 |
| Directly compressible excipient comprising mannitol and calcium silicate | 52 |
| Starch, Ph.Eur. | 8.0 |
| Microcrystalline cellulose, USP | 11.0 |
| Crospovidone, USP | 5.0 |
| Aspartame, UP | 3.0 |
| Acesulfame K, Ph.Eur. | 3.0 |
| Colloidal silicon dioxide, USP | 1.0 |
| Grapes Flavor | 1.0 |
| Magnesium stearate, USP | 1.0 |
| **Total** | **100.0** |

Memantine was dissolved in water and to this solution a cationic ion exchange resin was added. The suspension was stirred for 3 hours and the complexed memantine was separated, dried and blended with other excipients in a blender to get a uniform mass except lubricant. The mass was lubricated and compressed into tablets having following parameters:

| | |
|---|---|
| Hardness (N) | : 20-40 |
| Friability (%) | : 0.66 % |
| Disintegration time (sec) | : 8-12 |
| Disintegration time in oral cavity (sec) | : 20-30 |

Tablets with desired taste masking, friability and disintegration time were obtained.

### Example 3: Taste-masking of memantine using coating

**Table 3: Composition of orally disintegrating tablet**

| **Ingredients** | **mg/ tablet** |
|---|---|
| Coated memantine equivalent to 5 mg | 22.0 |
| Directly compressible excipient comprising mannitol and calcium silicate | 84.0 |
| Microcrystalline cellulose, USP | 75.0 |
| Crospovidone, USP | 12.0 |
| Aspartame, USP | 3.0 |
| Colloidal silicon dioxide, USP | 1.5 |
| Orange Flavor | 1.0 |
| Magnesium stearate, USP | 1.5 |
| **Total** | **200.0** |

Memantine was layered on non-pariel seeds and this drug loaded pellets were further coated with a combination of ethyl cellulose and hydroxypropyl methylcellulose (20:80) to a weight gain of 10%. Coated memantine pellets were blended with other excipients in a blender to obtain a uniform mass. This mass was lubricated and compressed into tablets having following parameters:

| | |
|---|---|
| Hardness (N) | : 45-60 |
| Friability (%) | : 0.48 % |
| Disintegration time (sec) | : 20-25 |
| Disintegration time in oral cavity (sec) | : 40-55 |

Tablets with desired taste masking, friability and disintegration time were obtained.

### Example 4: Taste-masking of memantine using cyclodextrin treatment

**Table 4: Composition of orally disintegrating tablet**

| **Ingredients** | **mg/ tablet** |
|---|---|
| Treated memantine equivalent to 10 mg | 35.0 |
| Directly compressible excipient comprising mannitol and calcium silicate | 70.0 |
| Starch, Ph.Eur. | 15.0 |
| Crospovidone, USP | 12.0 |
| Aspartame, USP | 4.4 |
| Colloidal silicon dioxide, USP | 1.3 |
| Orange Flavor | 1.0 |
| Magnesium stearate, USP | 1.3 |
| **Total** | **140.0** |

Memantine was mixed with beta cyclodextrin (1:2.5) in a ball mill for about 6 hours. Such treated memantine was then incorporated into a orally disintegrating tablet base. Cyclodextrin treated memantine was blended with other excipients in a blender to obtain a uniform mass. This mass was lubricated and compressed into tablets having following parameters:

| | |
|---|---|
| Hardness (N) | : 35-50 |
| Friability (%) | : 0.59 % |
| Disintegration time (sec) | : 15-20 |
| Disintegration time in oral cavity (sec) | : 30-45 |

Tablets with desired taste masking, friability and disintegration time were obtained.

### Example 5: Memantine melt extruded pellets coated using Kollicoat SR 30 D

**Table 5: Melt extruded pellets in orally disintegrating tablets**

| **Ingredients** | **Quantity(mg/tab)** |
|---|---|
| **a. Preparation of melt extruded pellets** | |
| Memantine hydrochloride | 5.0 |
| Microcrystalline cellulose | 15.0 |
| Glyceryl behenate USP | 20.0 |
| Dibutyl phthalate, USP | 2.0 |

| **b. Coating using Kollicoat SR 30 D** | |
|---|---|
| Memantine pellets | 42.0 |
| Kollicoat SR 30 D | 8.4 |

| **b. Formulation of Memantine tablet** | |
|---|---|
| Coated memantine pellets | 50.4 |
| Directly compressible excipient comprising mannitol and calcium silicate | 48.0 |
| Microcrystalline cellulose, USP | 16.0 |
| Crospovidone, USP | 6.0 |
| Aspartame, USP | 3.6 |
| Acesulfame K, Ph.Eur. | 3.0 |
| Colloidal silicon dioxide, USP | 1.0 |
| Magnesium stearate, USP | 1.0 |
| Peppermint Flavor | 1.0 |
| **Total** | **130.0** |

Memantine beads of controlled size and density using the extrusion technique was prepared by passing / forcing a concentrated formulation of memantine and glyceryl behenate through a fine nozzle. The extruded mass was then cut and shaped, in a spheronization process, to produce beads suitable for formulation as controlled release multiparticulates. The resultant beads were further coated with Kollicoat SR 30 D to a weight gain of 20% by weight for additional release rate control and taste masking. These taste masked memantine beads was mixed with other excipients and compressed into orally disintegrating tablets. Tablets with desired taste and disintegration time were obtained.

### Example 6: Memantine complexation with saccharin sodium

**Table 6: Composition of orally disintegrating memantine tablets**

| **Ingredients** | **mg/ tablet** |
|---|---|
| Memantine complex equivalent to 5 mg | 10.0 |
| Directly compressible excipient comprising mannitol and calcium silicate | 54.0 |
| Starch, Ph.Eur. | 14.0 |
| Crospovidone, USP | 12.0 |
| Aspartame, USP | 4.4 |
| Colloidal silicon dioxide, USP | 1.3 |
| Grapes Flavor | 1.0 |
| Magnesium stearate, USP | 1.3 |
| **Total** | **100.0** |

Memantine hydrochloride was dissolved in water reacted with saturated solution of sodium saccharin. This reaction produced salt of memantine saccharate which was incorporated in orally disintegrating tablets of memantine. Tablets with desired taste and disintegration time were obtained.

### Example 7: Orally Disintegrating Tablets of Memantine and Donepezil

**Table 7: Composition of orally disintegrating tablets**

| **Ingredients** | **mg/ tablet** |
|---|---|
| Memantine hydrochloride | 5.0 |
| Donepezil hydrochloride | 5.0 |
| Eudragit EPO, USP | 10.0 |
| Directly compressible excipient comprising mannitol and calcium silicate | 64.0 |
| Starch, Ph.Eur. | 10.0 |
| Microcrystalline cellulose, USP | 24.0 |
| Crospovidone, USP | 12.0 |
| Aspartame, USP | 3.0 |
| Acesulfame K, USP | 3.0 |
| Colloidal silicon dioxide, USP | 1.5 |
| Peppermint Flavor | 1.0 |
| Magnesium stearate, USP | 1.5 |
| **Total** | **140.0** |

Memantine and donepezil were taste masked using Eudragit EPO and blended with directly compressible excipient along with other excipients to compress orally disintegrating tablets.

### Orally disintegrating tablets exhibited following parameters:

| | |
|---|---|
| Hardness (N) | : 25-55 |
| Friability (%) | : 0.24 % |
| Disintegration time (sec) | : 10-12 |
| Disintegration time in oral cavity (sec) | : 22-28 |

## Claims

1. An orally disintegrating tablet composition of memantine or a pharmaceutically acceptable salt thereof, comprising (a) memantine or a pharmaceutically acceptable salt thereof; (b) at least one taste-masking agent; and (c) at least one pharmaceutically acceptable excipient.

2. The tablet composition of claim 1 wherein the taste-masking agent is ion exchange resin, water insoluble excipient, cyclodextrin, sugar substitute for salt formation, sweetener, flavor or a combination thereof.

3. The tablet composition of claim 2 wherein the taste-masking agent is an ion exchange resin.

4. The tablet composition of claim 3 wherein said ion exchange resin is a cationic ion exchange resin.

5. The tablet composition of claim 2 wherein the taste-masking agent is at least one water insoluble excipient.

6. The tablet composition of claim 5 wherein said water insoluble excipient is vinyl chloride, vinyl acetate, vinyl carbonate, methacrylic acid, a polymethacrylic acid copolymer, other polymethylmethacrylates, ethyl cellulose, nitrocellulose, acrylonitrile- styrene copolymer, vinylidene chloride-acrylonitrile copolymer, polyethylene, polyethylene oxide, polystyrene, ethylene vinyl acetate, cellulose acetate, cellulose acetate phthalate, cellulose acetate butyrate, hydroxypropylmethylcellulose phthalate, Cutina (hydrogenated castor oil), Hydrobase (hydrogenated soybean oil), Castorwax (hydrogenated castor oil), Croduret (hydrogenated castor oil), Carbowax, Compritol (glyceryl behenate), Sterotex (hydrogenated cottonseed oil), Lubritab (hydrogenated cottonseed oil), Apifil (wax yellow), Akofine (hydrogenated cottonseed oil), Softtisan (hydrogenated palm oil), Hydrocote (hydrogenated soybean oil), Corona (lanolin), Gelucire (macrogolglycerides lauriques), Precirol (glyceryl palmitostearate), Emulcire (cetyl alcohol), Plurol diisostearique (polyglyceryl diisostearate), Geleol (glyceryl stearate), hydrogenated palm oil, hydrogenated castor oil, hydrogenated cottonseed oil, stearic acid, palmitic acid, cetyl alcohol, or stearyl alcohol, or a mixture thereof.

7. The tablet composition of claim 6 further comprising at least one water soluble excipient, said water soluble excipient being polyvinylpyrrolidone, hydroxypropylmethylcellulose, polyethylene glycol, poloxamer, hydroxypropyl cellulose, lactose, mannitol, methylcellulose, guar gum, xanthan gum, gum arabic, tragacanthan, or hydroxyethyl cellulose, or mixtures thereof.

8. The tablet composition of claim 2 wherein the taste-masking agent is cyclodextrin or its derivatives.

9. The tablet composition of claim 2 wherein the taste-masking agent is a sugar substitute for salt formation with memantine or pharmaceutically acceptable salts, said sugar substitute being saccharin sodium, cyclamate, or acesulfame, or a mixture thereof.

10. The tablet composition of claim 1 wherein said excipient is a directly compressible excipient, binder, disintegrant, superdisintegrant, diluent, salivating agent, surfactant, flavor, sweetener, colorant, souring agent, viscosity builder, glidant, lubricant, solubilizer, or stabilizer.

11. The tablet composition of claim 10 wherein said directly compressible excipient comprises at least one water soluble excipient and calcium silicate, said water soluble excipient being a carbohydrate, a water soluble salt, or a polyhydric alcohol or a derivative thereof.

12. The tablet composition of claim 10 wherein said carbohydrate is a monosaccharide, disaccharide, oligosaccharide or a polysaccharide, said disaccharide being mannitol.

13. The tablet composition of claim 1 wherein the *in vitro* disintegration time is less than 60 seconds.

14. The tablet composition of claim 1 further comprising an additional active agent, said active agent being donepezil, rivastigmine, metrifonate, tacrine, amantadine, galantamine, olanzapine, piracetam, aniracetam, nefiracetam, oxiracetam, lazabemide, selegiline, amitriptyline, clomipramine, trimipramine, doxepin, nortriptyline, desipramine, protriptyline, fluoxetine, paroxetine, citalopram, sertraline, fluvoxamine, escitalopram, reboxetine, atomoxetine, venlafaxine, milnacipran, or duloxetine.

15. The tablet composition of claim 1 wherein said composition readily disintegrates in the oral cavity within about 60 seconds, releasing controlled release, taste-masked memantine.
